# EUROPÄISCHE PATENTANMELDUNG

(11) **EP 2 120 049 A1**
(43) Veröffentlichungstag der Anmeldung: **18.11.2009**
(21) Anmeldenummer: 08008903.0
(22) Anmeldetag: 14.05.2008
(51) Int. Cl.: G01N 33/68

(54) **Lösliches Thrombomodulin als Biomarker bei Therapien mit Gerinnungshemmern**

(71) Anmelder: PAION Deutschland GmbH, 52062 Aachen (DE)
(72) Erfinder: Gillen, Clemens, 52159 Roetgen (DE); Öhlin, Ann-Kristin, Lund University, University Hospital, 221 85 Lund (SE)
(74) Vertreter: Von Renesse, Dorothea

(57) **Zusammenfassung**

Ein in vitro-Verfahren zur Auswahl eines für die Therapie mit Gerinnungshemmern geeigneten Patienten, umfassend folgende Schritte:
e. Entnahme einer Probe aus einer Körperflüssigkeit, -zellen oder -gewebe aus dem Patienten,
f. Ermittlung des Iöslichen Thrombomodulins in der entnommenen Probe,
g. Vergleich der ermittelten Konzentration oder Aktivität des löslichen Thrombomodulins in der Probe mit einem Standardwert,
h. Auswahl desjenigen Patienten, dessen Probe im Vergleich zu dem Standardwert keine wesentlich erhöhte Konzentration oder Aktivität an löslichem Thrombomodulin aufweist für eine Behandlung mit Gerinnungshemmern.

## Beschreibung

Die Erfindung betrifft die Verwendung eines Biomarkers zur Identifikation von Patienten, die für eine Therapie mit gerinnungshemmenden Medikamenten geeignet sind.

Verletzungen der Endothelzellen, die die Blutgefäße auskleiden, führen in der Regel zur Blutstillung (Hämostase), die über eine komplexe Abfolge molekularer Interaktionen, die sogenannte Gerinnungskaskade, vermittelt wird. Diese Kaskade kulminiert in der Bildung von unlöslichem Fibrin aus löslichem Fibrinogen, welches zusammen mit Thrombozyten einen Blutpfropf (Thrombus) bildet, der den weiteren Blutverlust hemmt. Nach der Hämostase erfolgt die Wundheilung, der Thrombus wird abgebaut und die Integrität des Blutgefäßes wird wieder hergestellt.

Während eine effiziente verletzungsverursachte Blutstillung überlebenswichtig ist, ist eine fehlgesteuerte Bildung von Thromben in arteriellen oder venösen Gefäßen hingegen eine häufige Ursache für schwere, zumeist akut auftretende Erkrankungen, die Invalidität oder Tod nach sich ziehen können.

Die Bildung von Thromben ist Ursache für Erkrankungen wie Schlaganfall, Herzinfarkt, tiefe Beinvenenthrombose oder Lungenembolie. Diese thrombotischen Erkrankungen, insbesondere Herz-Kreislauf-Erkrankungen (hautsächlich Herzinfarkt) und Schlaganfall, stellen die häufigste bzw. dritthäufigste Todesursache dar.

Die Bildung von Thromben ist aber nicht nur Ursache von Erkrankungen sondern es gibt eine Reihe von Erkrankungen, die mit einem erhöhten Thromboserisiko einhergehen, oder im Spätstadium zu einer Thrombusbildung führen, wie z.B. Schlaganfall, eine transitorische ischämische Attacke, Herzinfarkt, labile Angina, Vorhofflimmern, Nierenschäden, disseminierte intravasale Koagulation, Sepsis oder Lungenembolie. Zudem verursacht eine Reihe von medizinischen Behandlungen in ein erhöhtes Thromboserisiko, insbesondere kardiovaskuläre Operationen wie Bypass-Operationen oder die perkutane transluminale koronäre Angioplastie, Implantation von Herzklappen oder Einsetzen von Stents. Die medikamentöse Behandlung von Patienten mit Thromboserisiko stellt dementsprechend eine der zentralen (Begleit)Maßnahmen bei der Behandlung dieser Erkrankungen dar, vor allem bei kardiovaskulären Erkrankungen, um nämlich thrombotische Ereignisse möglichst zu vermeiden.

Die Behandlung eines Patienten mit Thromboserisiko mit Vitamin K-Antagonisten (VKA) ist zur primären und sekundären Prävention thromboembolischer Ereignisse weitgehend akzeptiert. Warfarin stellt den bei weitem am häufigsten verwendeten VKA dar und gehört zu den 10 am meisten verwendeten Medikamenten überhaupt.

Die gravierendste ungewünschte Nebenwirkung bei einer Antikoagulanz-Behandlung liegt in einem erhöhten Blutungsrisiko. So wurde bei einer Patientenpopulation mit Warfarin-Behandlung in Nordschweden das Risiko für klinisch relevante Blutungen mit 3.9% aller Patienten pro Behandlungsjahr ermittelt. In einer Studien-Metaanalyse wurde bei oralen Antikoagulantien (hauptsächlich Warfarin) für schwere Blutungen eine Rate von 7.22 % pro Patientenjahr ermittelt, die Rate tödlicher Blutungen lag bei 1.31 % pro Patientenjahr (Linkins et al., Ann. Intern. Med. (2003), 139: 893-900).

Es werden daher seit langem große Anstrengungen unternommen, um die Sicherheit dieser Antikoagulantien zu überwachen, hierbei vor allem um das Blutungsrisiko zu minimieren. In der gegenwärtigen VKA-Therapie wird die Dosis dazu engmaschig überwacht, indem nämlich regelmäßig bei den Patienten nach einer Blutentnahme die Prothrombinzeit als "international normalized ratio" (INR) bestimmt wird. Dabei handelt es sich um ein weltweit in Laboratorien standardisiertes Verfahren zur Prüfung des Zustandes des extrinsischen Systems der Blutgerinnung. Dieser Wert liefert aber nur einen groben Anhaltspunkt für das Blutungsrisiko. So sind selbst bei einem empfohlenen INR-Bereich von 2-3 noch 2-3% schwere Blutungen zu beobachten, und schon bei einer mäßigen Erhöhung des INR-Wertes auf 3-4 steigt das Blutungsrisiko um etwa 50%.

Antikoagulantien wie z.B. Warfarin wirken bei Patienten sehr unterschiedlich. Hierbei spielen auch genetische Unterschiede eine Rolle. So konnte gezeigt werden, dass Polymorphismen im VKORC1-Gen und im Gen der Cytochrom P450 Oxidase Subtyp C9 mit der Warfarin-Wirkung korrelieren.

Obwohl in der Vergangenheit zahlreiche Anstrengungen unternommen wurden, das Blutungsrisiko unter Antikoagulanzbehandlung vorherzusagen oder doch wenigstes abzuschätzen - und davon ausgehend bestimmte Patienten von der weiteren Behandlung auszuschließen - sind bisher nur wenige Risikofaktoren beschrieben worden, die eben diese gesuchte Vorhersage ermöglichen. So gelten z.B. das Alter der Patienten, frühere gastrointestinale Blutungen und ein Bluthochdruck als Risikofaktoren für Blutungen.

Diese Risikofaktoren sind aber ebenso Risikofaktoren für thromboembolische Ereignisse und daher für die Abwägung des Risiko-Nutzen-Verhältnisses einer Antikoagulanzbehandlung kaum von Wert. So deutet nämlich beispielsweise der Risikofaktor Bluthochdruck" einerseits auf ein erhöhtes Blutungsrisiko hin - was gegen den Beginn oder die Weiterführung einer Antikoagulanzbehandlung spricht - und zeigt andererseits auch ein erhöhtes Thromboserisiko an. Dies spricht jedoch für eine Antikoagulanzbehandlung. Die Bestimmung des Risikofaktors "Bluthochdruck" wird dem behandelnden Arzt daher in seiner schwierigen Entscheidung über den Beginn oder die Weiterführung einer Behandlung mit Gerinnungshemmern nicht helfen. Somit ist der Bluthochdruck kein geeigneter Biomarker.

Es besteht daher ein Bedarf an spezifischen Biomarkern, die es erlauben, Patienten zu identifizieren, die eine geringe Blutungsneigung bei gleichzeitigem Risiko für thromboembolische Ereignisse besitzen. Diese können anschließend aufgrund ihrer geringen Blutungsneigung eine Therapie durchlaufen, die die Gerinnungsneigung beeinflußt, insbesondere die Gerinnung kennt, und müssten nicht zur sicheren Vermeidung einer Blutung von derartigen Behandlungen ausgeschlossen werden.

Es ist somit die Aufgabe der vorliegenden Erfindung, ein Verfahren bereitzustellen, das diejenigen Menschen oder Tiere identifiziert, die im Hinblick auf ihr individuelles Blutungsrisiko mit einem vertretbaren Risiko/Nutzen-Verhältnis mit Gerinnungshemmern behandelbar sind. Dies gilt insbesondere für Antikoagulantien, die die Blutungsneigung des Patienten erhöhen.

Diese Aufgabe wird durch die direkte oder indirekte Ermittlung des löslichen Thrombomodulins (soluble thrombomdulin, sTM) in Proben aus Gewebe, Zellmaterial oder Körperflüssigkeiten eines Patienten gelöst, da über die Ermittlung des sTM Spiegels die Blutungsneigung spezifisch abschätzbar ist. Somit kann der individuelle, d.h. der dem jeweiligen Patienten eigene sTM Wert als Biomarker herangezogen werden, der es erlaubt zu entscheiden, ob der Patient tatsächlich für eine Behandlung mit Gerinnungshemmern geeignet ist, oder aber ein erhöhtes Blutungsrisiko der Aufnahme oder der Weiterführung dieser Therapie entgegensteht.

Die Erfindung erlaubt es, Patienten in Abhängigkeit von dem in der jeweiligen Körperflüssigkeit, Zell- oder Gewebeprobe ermittelten sTM-Spiegel in verschiedene Patientenpopulationen aufzuteilen, und diese anschließend unterschiedlich zu behandeln. Die Individuen mit wesentlich erhöhtem sTM-Spiegel stellen eine Patientengruppe dar, die keine Therapie mit Gerinnungshemmern erhalten sollten, die das bereits erhöhte Blutungsrisiko noch weiter erhöhen. Individuen mit normalem, d.h. gegenüber dem "gesunden" Zustand nicht wesentlich erhöhtem sTM-Spiegel stellen hingegen eine Patientenpopulation dar, die mit Therapien behandelbar ist, die mit einer Erhöhung des Blutungsrisikos einhergehen. In Abhängigkeit von dem jeweils ermittelten sTM-Spiegel kann außerdem die individuell verabreichte Dosierung von Gerinnungshemmern auf den jeweiligen Patienten angepasst werden. Daher ist die Bereitstellung individuell angepasster Dosiseinheiten möglich.

Bisher war es nicht möglich, die Ermittlung des sTM-Spiegels zu dem erfindungsgemäßen Zweck einzusetzen, da man davon ausging, dass der sTM-Spiegel gleichzeitig mit einem erhöhten Thromboserisiko korreliert. Die Ermittlung des sTM-Spiegels hatte daher gegenüber den klassischen Risikofaktoren wie Alter oder Bluthochdruck keinerlei Erkenntnisgewinn, der eine Differenzierung zwischen den Patienten erlaubte. Als Biomarker im Sinne der vorliegenden Erfindung schien das sTM somit nicht geeignet zu sein.

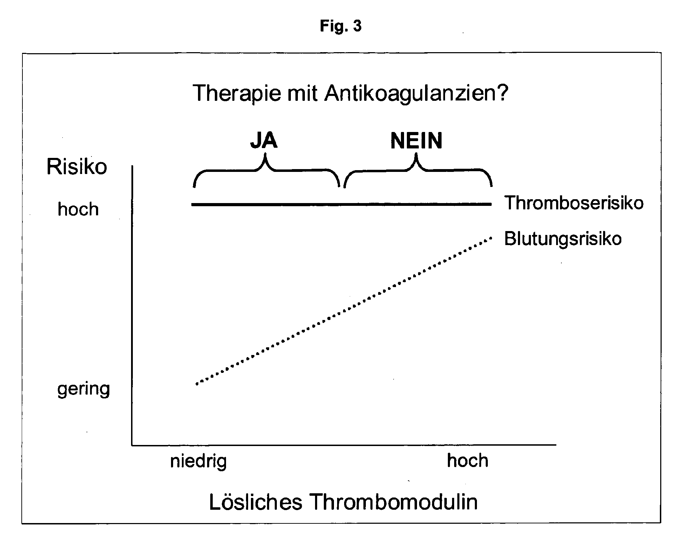

Nun haben die Erfinder aber in einer prospektiven Studie an VKA-behandelten Patienten erkannt, dass ein erhöhter Plasmaspiegel an löslichem Thrombomodulin zwar mit sowohl schweren als auch klinisch relevanten Blutungen korreliert. Allerdings wurde keine Korrelation zwischen dem sTM-Spiegel und nicht-tödlichen kardiovaskulären Ereignissen oder der Gesamt-Todesrate gefunden (siehe Abbildung 3).

Die Korrelation zwischen erhöhtem sTM-Spiegel und Blutungsrisiko war aber - wie schon oben ausgeführt - bereits früher aufgrund klinischer Studien vermutet worden (Jansson et al., Circulation (1997), 96: 2938-43, oder van der Heijden et al., J. Thromb. Haemost. (2004), 2:1104-1109). Die Bedeutung der neuen und überraschenden Erkenntnisse der Erfinder liegt daher vor allem darin, erkannt zu haben, dass ein erhöhter sTM-Spiegel nicht mit einem erhöhten Thromboserisiko (ermittelt z.B. durch kardiovaskuläre Ereignisse und Gesamt-Todesfälle) einhergeht.

Erst diese Erkenntnis erlaubt es, lösliches Thrombomodulin erfindungsgemäß als Biomarker für das individuelle Blutungsrisiko einzusetzen, da es erst jetzt das bisherige "diagnostische Dialemma" des Arztes aufhebt. Bisher war der Nachweis eines erhöhten sTM-Spiegels für den Arzt ein Hinweis, der für den Einsatz gerinnungshemmender Arzneimittel sprach. Gleichzeitig sprach das mit dem erhöhten sTM-Spiegel verbundene Blutungsrisiko jedoch *gegen* den Einsatz gerinnungshemmender Stoffe.

Die Ergebnisse, die zu der erfindungsgemäßen Verwendung von sTM führten, waren für die Fachwelt überraschend, weil eine Vielzahl von Publikationen und Studienergebnissen eine vermeintliche Bedeutung des erhöhten sTM-Plasmaspiegel als Hinweis auf ein Thromboserisiko zeigte. Dies wurde damit erklärt, dass ein erhöhter sTM Spiegel durch die vermehrte proteolytische Abspaltung des membrangebundenen Thrombomodulins zurückzuführen sei. Der erhöhte sTM-Spiegel schien daher mit einer Endothelschädigung verbunden zu sein. Außerdem wurde angenommen, dass die Abnahme des Thrombomodulins auf der Oberfläche von Endothelzellen die Entstehung von Thrombosen begünstigen könnte (Ishii et al., Thromb. Haemost. (1991), 65: 618-623; Takano et al., Blood (1990), 76: 2024-2029).

Übereinstimmend mit dieser Hypothese wurde eine Assoziation zwischen einem erhöhten sTM-Spiegel mit rezidivierendem Myokardinfarkt und der peripheren arteriellen Erkrankung gefunden (Blann et al., Eur. J. Haematol. (1997), 59: 115-120; Blann and McCollum, Blood Coagul. Fibrinolysis (1999), 10: 375-380). Ein erhöhter sTM-Spiegel gilt ebenso als Marker für die Schädigung von Endothelzellen.

Auch bei anderen Erkrankungen wurde ein erhöhter sTM-Spiegel gefunden und als Marker der Endothelzellschädigung interpretiert, wie zB. bei der thrombotischthrombozytopänischen Purpura (Dittmann und Majerus, Blood (1990), 75: 329-336; Takahashi et al., Am. J. Hematol. (1991), 38: 174-177), der diabetischen Mikroangiopathie (Tanaka et al., Clin.Chem. (1991), 37: 269-272), der atheromen arteriellen Erkrankung (Seigneur et al., Thromb. Res. (1993), 71: 423-431), der disseminierten intravasalen Koagulation (DIC; Verbrauchskoagulopathie; Takahashi et al., Am. J. Haematol. (1992), 41:32-39) und bei vaskulären Kollagenerkrankungen (Ohdama et al., Am. J. Clin. Path. (1994), 101: 109-113).

Die vorliegende Erfindung stellt somit ein Verfahren bereit, das durch eine einfache in *vitro*-Messung diejenigen Patienten identifiziert, die vorteilhaft mit Gerinnungshemmern behandelbar sind. Dies gilt insbesondere für eine Antikoagulanzbehandlung. Dazu kann nach der Probenentnahme beim Patienten in der Probe die Konzentration und/oder die Aktivität an löslichem Thrombomodulin direkt oder indirekt bestimmt, die resultierenden Werte mit den Werten einer Kontrollgruppe ("Standardwert") verglichen, und die Patienten, die gegenüber dem Standardwert keine oder keine wesentlich erhöhte Konzentration und/oder Aktivität an löslichem Thrombomodulin aufweisen, für eine gerinnungshemmende Therapie vorgesehen werden. Ebenso ist die Anpassung der Dosis eines gerinnungshemmenden Mittels an die jeweils individuell ermittelten sTM-Werte möglich. Damit erlaubt die Erfindung eine individuelle Optimierung des Dosierungsplans des jeweils eingesetzten Gerinnungshemmers sowie gegebenenfalls die Auswahl des Gerinnungshemmers und des Applikationsschemas.

Zur Ermittlung des Standardwertes können Patienten oder Probanden herangezogen werden, die entweder mit Gerinnungshemmern behandelt werden oder aber auch keine Gerinnungshemmer erhalten. Die ermittelten sTM-Werten können dann beispielsweise in drei Tertilen eingeteilt werden. Die erste Tertile enthält etwa das Drittel mit den geringsten sTM-Werten, die zweite Tertile das Drittel mit den mittleren sTM-Werten und die dritte Tertile das Drittel mit den höchsten sTM-Werten. Als Standardwert im Sinne dieser Erfindung kann der bei dieser Kontrollgrupe ermittelte Mittelwert der ersten Tertile herangezogen werden. Eine "nicht wesentliche" Erhöhung (im Sinne der Erfindung) kann demnach ein sTM-Wert der Probe sein, der bei der Einteilung der Kontrollgruppe in Tertilen in die erste Tertile einzuordnen ist.

Alternativ kann ein Standratwert herangezogen werden, der etwa 80% (z.B. 83%) des arithmetischen Mittelwertes der gesamten Kontrollgruppe entspricht.

Eine "nicht-wesentliche" Erhöhung des sTM-Wertes ist vorteilhafterweise eine Erhöhung des Standardwertes um bis zu etwa 30 %, bevorzugt um bis zu etwa 20, oder 10%, besonders bevorzugt um unter 5%, beispielsweise um etwa 2%.

Die Kontrollgruppe sollte möglichst im Alter mit den untersuchten Patienten vergleichbar sein. Es kann dabei vorteilhaft sein, dass die Probanden/Patienten auch in anderen Parametern mit dem erfindungsgemäß untersuchten Patienten vergleichbar sind, so z.B. in der Ko-Medikation (insbesondere bezüglich Kontrazeptiva), in dem Alkohol- oder Nikotingebrauch, im Geschlecht oder in Mutationen / Polymorphismen in gerinnungsrelevanten Genen.

In einer Ausführungsform der vorliegenden Erfindung werden diejenigen Patienten für eine spätere Behandlung mit gerinnungshemmenden Mitteln ausgewählt, deren Konzentration von löslichem Thrombomodulin im Blutplasma weniger als etwa 6,1 ng/ml, bevorzugt weniger als etwa 5,5 ng/ml und besonders bevorzugt weniger als etwa 5,0 ng/ml beträgt.

Es ist dem Fachmann bekannt, dass alle klinisch angewendeten oralen oder parenteralen Gerinnungshemmer (so auch Antikoagulantien) ein gewisses Blutungsrisiko nach sich ziehen, und dass auch die in der Entwicklung befindlichen Antikoagulantien angesichts der Komplexität des Gerinnungssystems und bei bevorzugter Anwendung in älteren oft komorbiden Patienten ein signifikantes Restrisiko für Blutungen verursachen werden.

Unter löslichem Thrombomodulin (soluble thrombomodulin; sTM) versteht man jegliche Thrombomodulinfragmente, die in Körperflüssigkeiten oder Gewebe gelöst vorkommen und/oder Abbauprodukte des zellmembrangebundenen Thrombomodulins darstellen. Im Sinne der Erfindung ist es dabei nicht erforderlich, daß das entsprechende Fragment die biologischen Aktivitäten des nativen TM zeigt.

Erfindungsgemäß kann nicht nur eine Probe aus dem Plasma zur Bestimmung des hier relevanten sTM-Spiegels herangezogen werden. So ist es nämlich bekannt, dass Thrombomodulin in löslicher Form (soluble Thrombomodulin, sTM) auch im Urin (Ishii and Majerus, J. Clin. Invest. (1985), 76: 2178-2181), in der Synovialflüssigkeit (Conway and Nowakowski, Blood (1993); 81: 726-733) oder in der Samenflüssigkeit nachweisbar ist (Lwaleed et al., Blood Coagul. Fibrinolysis (2008), 19:142-145). Die jeweiligen Werte des sTM können dabei im Bereich von etwa 3.2ng/ml bis 35ng/ml liegen. Dieser Wert gilt auch für das Plasma. Diese Werte können nach Ermittlung der Tertilen oder als arithmetische Mittelwerte als Standardwerte für den Vergleich mit dem sTM-Spiegel im Patienten herangezogen werden.

Die Ermittlung des löslichen Thrombomodulins kann durch direkte oder indirekte Methoden erfolgen. Unter einer direkten Methode versteht man ein analytisches Verfahren, bei dem das lösliche Thrombomodulin unmittelbar selber nachgewiesen wird. Diese direkte Messung kann die Menge des löslichen Thrombomodulins und/oder die Aktivität des löslichen Thrombomodulins erfassen. Beispiele für eine direkte Methode zur Messung der Proteinmenge sind eine Western-Blot-Analyse und/oder ein ELISA-Test. Beispiele für eine direkte Methode zur Erfassung der Proteinaktivität sind ein in *vitro*-Testverfahren und/oder ein *vitro*-Verfahren unter Benutzung von menschlichen, tierischen, Pflanzen-, Insekten-, Bakterien- oder Hefezellen.

Unter einer indirekten Methode versteht man ein analytisches Verfahren, bei dem nicht das lösliche Thrombomodulin selber, sondern ein andere Substanz nachgewiesen wird, die in einem gesetzmäßigen Zusammenhang mit dem löslichen Thrombomodulin steht.

Indirekte Methoden können z. B. die komplementäre Bestimmung des membrangebundenen Thrombomodulins in einer Gewebeprobe sein. Des Weiteren kann die Aktivität oder der Spiegel an natürlichen Substraten des Thrombomodulins im Plasma oder Gewebeproben bestimmt werden. Als Beispiele seien hier das aktivierte Protein C (APC), der aktivierte Thrombin-aktivierbare Fibrinolyse-Inhibitor (TAFla) oder der Plasminogen Aktivator Inhibitor 1 (PAI-1) genannt. Auch Substrate dieser Substrate können einen Rückschluss auf die sTM-Menge geben, wie z.B. die Menge des durch APC aktivierten Protease-aktivierbaren Rezeptors Subtyp 1 (PAR-1). Da sTM ebenso wie das membrangebundene Thrombomodulin Thrombin modulieren kann, sind auch Methoden zur Bestimmung der Thrombingenerierung geeignet, das sTM zu bestimmen. Als Beispiele für einen Thrombingenerierungsassay seien hier der CAT-Assay (Fa. Thrombinoscope, Maastricht, NL), der Technothrombin^{®} TGA Assay (Fa. Technoclone, Wien, Österreich), der Endogenous Thrombin Potential Assay (ETPa) (Fa. Siemens Healthcare Diagnostics, Erlangen, Deutschland) oder der Pefakit^{®} Thrombin Dynamics Test (inTDT) (Fa. Pentapharm, Basel, Schweiz) genannt. Ebenso könnte man die endogenen Substanzen bestimmen, die ursächlich für einen erhöhten sTM-Spiegel verantwortlich sind, wie z.B. die neutrale Endolase oder die RHBDL2-ähnliche Rhomboid-Protease. Schließlich könnte man auch diejenigen Substanzen, insbesondere Proteasen, ermitteln, die im Körper für eine Weiterprozessierung oder einen Abbaus des löslichen Thrombomodulins verantwortlich sind, wie z.B. das Cathepsin G oder die neutrophile Elastase und weiteren Substanzen, die bei akuten oder chronischen Entzündungsprozessen in dem vorgenannten Sinne aktiv sind.

Bei der Bestimmung des Thrombomodulins über eine indirekte Methode kann man durch Voruntersuchungen die Menge des löslichen Thrombomodulins über einen Umrechnungsfaktor herleiten. So ist bekannt, dass in den in vitro angewendeten Thrombingenerierungsassay einige Parameter wie die Hemmung des endogenen Thrombinpotentials ("ETP") oder die Hemmung der Höhe der Thrombingenerierungskurve ("Thrombin-Peak") proportional zu der Konzentration des in der Probe vorhandenen löslichen Thrombomodulins ist. Über die Verwendung des löslichen Thrombomodulins als Standard zur Erstellung einer Eichkurve kann von den Ergebnissen des Thrombingenerierungsassays dementsprechend auf die in der der Probe vorhandene Menge an löslichem Thrombomodulin geschlossen werden.

Die Erfindung eignet sich besonders für die Ermittlung von Patienten für eine anschließende Behandlung mit Gerinnungshemmern, die an einer Erkrankung leiden, die ein erhöhtes Thromboserisiko mit sich bringt, wie beispielsweise Schlaganfall, transitorische neurologischen Attacken (TNA), transitorische ischämische Attacken (TIA), Myokardinfarkt, das prolongierte reversible ischämisch-neurologische Defizit (PRIND), labile Angina, Vorhofflimmern, Nierenschäden, disseminierte intravasale Koagulation, Sepsis, Lungenembolie oder tiefe Beinvenenthrombosen.

Die Erfindung unterstützt oder ermöglicht somit eine Entscheidung über die Anwendbarkeit einer Therapie mit Gerinnungshemmern, wie eine AntikoagulanzTherapie bei einem Patienten oder aber deren individuelle Anpassung. Bei den dafür einsetzbaren Gerinnungshemmern handelt es sich im Sinne der Erfindung bevorzugt um Wirkstoffe, die die Blutungsneigung oder das Blutungsrisiko im Patienten erhöhen. Diese Wirkung kann durch die Veränderung der Menge oder Aktivität an Gerinnungsfaktoren und anderen Blutbestandteilen hervorgerufen werde, aber auch durch Veränderungen im Blutdruck, Blutviskosität, oder Blutflußgeschwindigkeit oder auch durch Veränderung der Blutgefäße, insbesondere der Endothelzellen. Insbesondere handelt es sich bei den Medikamenten um parenterale oder orale Antikoagulantien, Blutprodukte und Gerinnungsfaktorenkonzentrate, Fibrinolytika oder Thrombozytenfunktionshemmer. Beispiele für orale Antikoagulanzien sind Dabigatran Etexilate, Rivaroxaban, und Vitamin-K Antagonisten.

Beispiele für parenterale Antikoagulanzien sind Heparine, darunter unfraktionierte Heparine, und niedermolekulare Heparine wie Certoparin, Dalteparin, Enoxaparin, Nadroparin, Reviparin und Tinzaparin, das Heparinoid Danaparoid, und Hirudin.

Beispiele für Fibrinolytika sind Streptokinase, Urokinase, t-PA, Reteplase, Tenecteplase und DSPA. Beispiele für Thrombozytenfunktionshemmer sind Acetylsalicylsäure, Dipyridamol, Thienopyridine wie Ticloplidin oder Clopidogrel, Glykoprotein-Ilb-Illa-Inhibitoren wie Abciximab, Eptifibatid oder Prostacyclinderivate wie Iloprost.

Weiterhin lässt sich die Erfassung des sTM als Biomarker zur Anpassung der Dosierung eines therapeutischen Wirkstoffs bei Patienten oder im Rahmen einer klinischen Studie anwenden. In einer Ausführungsform wird die Dosierung 24-36 Stunden nach Gabe des Gerinnungshemmers (z.B. Warfarin) durch Bestimmung des INR überprüft, wobei das INR einem Bereich zwischen 2 und 3 liegen sollte. Die übliche Warfarin-Dosis beträgt 4-6 mg/Tag, wobei eine inverse Korrelation zum Alter der Patienten besteht. In 50-jährigen Patienten beträgt die durchschnittliche tägliche Dosis 6.3 mg, in 70-jährigen Patienten hingegen 3.6 mg. Darüber hinaus lässt sich der Biomarker zur Identifizierung von an klinischen Versuchen teilnehmenden Individuen, seien es gesunde Probanden oder Patienten, einsetzen.

In einem optionalen Schritt kann die nachfolgende Dosierung des Gerinnungshemmers an Patienten durch die Kombination mit weiteren Biomarkern optimiert werden. So sind für das Arzneimittel Warfarin Polymorphismen im VKORC1-Gen und im CYP2C19-Gen beschrieben worden, die zur Dosisfindung eingesetzt werden können und somit in Verbindung mit dem ermittelten sTM-Spiegel zu einer weiteren Optimierung der Dosierung dienen können.

Ebenso lässt sich sTM als Biomarker im Sinne der vorliegenden Erfindung zur Bewertung der Toleranz, Sicherheit und Wirksamkeit eines pharmazeutischen Wirkstoffes bei einem bestimmten Individuum oder bei der Identifizierung des Individuums, welches für die jeweilige Behandlung einer thromboembolischen oder kardiovaskulären Erkrankung in Frage kommt, sei es als eigenständiger Biomarker oder als Teil eines Tests einsetzen.

Das erfindungsgemäße Verfahren kann ebenso zur Entscheidungsfindung oder Anpassung einer nicht-medikamentösen Therapieform, insofern diese mit der Gabe von Gerinnungshemmern einhergeht, eingesetzt werden. Als Bespiele seien Prothesen, wie beispielsweise künstliche oder biologische Herzklappen, Transplantate wie beispielsweise Herz- oder Lebertransplantate, Implantate wie beispielsweise Stents, Herzschrittmacher oder Herzimplantate, physikalische Methoden zur Thrombusentfernung wie z. B. neurovaskuläre Retriever (Merci-Retriever), Ultraschall oder Mikroglasbläschen genannt. Dazu gehören auch Maschinen, die im kardiovaskulären Bereich eingesetzt werden, wie z.B. die Herz-Lunge-Maschine oder die extrakorporale Membranoxygenierungs-Maschine.

In dem erfindungsgemäßen Verfahren kann als Kontrolle bei der Konzentrationsbestimmung oder Aktivitätsbestimmung Thrombomodulin, bevorzugt lösliches Thrombomodulin oder besonders bevorzugt Solulin (siehe unten) verwendet werden. Thrombomodulin ist im Sinne der vorliegenden Erfindung als Protein definiert, inklusive aller Thrombomodulin-Modifikationen, Analoga, Fragmente oder Derivate hiervon, das im Wesentlichen die biologische Aktivität von Thrombomodulin besitzt, insbesondere nämlich einen funktionellen Komplex mit Thrombin bilden kann. Ein funktioneller Komplex mit Thrombin ist ein Komplex, der den Protein C/Protein S Signalweg aktivieren kann.

Das 6EGF-Fragment des Solulins ist ein Beispiel für ein Thrombomodulin-Fragment, das im Wesentlichen die gleiche biologische Aktivität aufweist, da es einen Komplex mit Thrombin ausbilden kann und dadurch Protein C aktivieren kann. Das 6EGF-Fragment besteht aus den sechs "Epithelial growth factor (EGF)"-ähnlichen Domänen des nativen Thrombomodulins.

Als "Iösliches Thrombomodulin" sind alle Thrombomodulin-Fragmente definiert, denen zumindestens die Transmembranregion fehlt, so daß das Molekül damit nicht mehr als Membranprotein vorliegt. Dem Fachmann sind zahlreiche Formen des löslichen Thrombomodulins bekannt, beispielsweise das sogenannte ART-123 (INN: Thrombomodulin alpha; Recomodulin™), das von der Firma Asahi Kasei Co. vertrieben wird oder das rekombinante lösliche humane Thrombomodulin Solulin (bisher kein INN vergeben), das von PAION Deutschland GmbH, Aachen entwickelt wird. Das rekombinante lösliche Thrombomodulin, bei dem gegenüber dem nativen humanen Thrombomodulin keine Veränderung innerhalb der Aminosäuresequenz vorliegt, ist Gegenstand der Patentschrift EP 0 312 598 B1.

Solulin ist ein lösliches, zudem Protease- und oxidationsresistentes Analogon des humanen Thrombomodulins und besitzt durch gezielte Entfernung der Glykosaminoglykankette eine lange Halbwertszeit im Körper. Solulin ist inter alia Gegenstand der europäischen Patentschriften EP 0 641 215 B1, EP 0 544 826 B1 als auch EP 0 527 821 B1. Bezüglich der strukturellen Eigenschaften der dort beschriebenen TM-Varianten werden diese Druckschriften vollumfänglich zu Zwecken der Offenbarung in Bezug genommen.

Solulin besitzt im Vergleich zu dem nativen humanen Thrombomodulin (SEQ. ID. No. 1) an folgenden Positionen Modifikationen: G -3V, Deletion der N-Terminalen Aminosäuren 1-3, M388L, R456G, H457Q, S474A und ein C-terminales Ende bei Aminosäure P490. Die Nummerierung entspricht dem in Sequenz No. 1 aufgelisteten nativen Thrombomodulin. Die Solulinsequenz (einschließlich ihrer Signalsequenz) als bevorzugte Ausführungsform der Erfindung ist in SEQ. ID. No. 2 wiedergegeben. Als Kontrolle in dem erfindungsgemäßen Verfahren wird aber vorzugsweise das "reife Protein" eingesetzt, das der SEQ. ID. No. 2 abzüglich der N-terminalen Aminosäuren 1 bis 18 entspricht.

Allerdings können im Rahmen der Erfindung auch Thrombomodulin-Analoga verwendet werden, die nur eines oder mehrere der oben aufgeführten Eigenschaften aufweisen, oder die in den oben erwähnten europäischen Patenten EP 0 544 826 B1, EP 0 641 215 B1 und EP 0 527 821 B1 erwähnten Eigenschaften besitzen.

Besonders bevorzugte Thrombomodulin-Varianten im Rahmen der Erfindung besitzen eine oder mehrere der im Folgenden aufgelisteten Eigenschaften:
(i) Sie sind oxidationsresistent,
(ii) Sie besitzen eine Prrotease-Resistenz,
(iii) Sie besitzen homogene N- oder C-Termini,
(iv) Sie liegen post-translational modifiziert vor, beispielsweise durch Glykosylierung an einigen der im nativen Thrombomodulin vorkommenden Glykosylierungsstellen (SEQ ID NO: 1),
(v) Sie besitzen lineare doppelt-reziproke Thrombinbindungseigenschaften,
(vi) Sie sind in wässrigen Lösungen bei relative geringen Mengen an Detergentien löslich, und besitzen typischerweise keine Transmembrandomäne,
(vii) Sie besitzen keine Glykosaminoglykan-Kette.

Die Herstellung dieser Analoga ist in den oben erwähnten europäischen Patenten offenbart.

Das besonders bevorzugte Molekül enthält sämtliche Modifikationen, und wird beispielweise durch Solulin repräsentiert.

In einer weiteren Ausführungsform der Erfindung können die in der Patentanmeldung WO 01/98352 und US 6632791 beanspruchten Thrombomoduline verwendet werden. Die Patentanmeldung WO 01/98352 beansprucht eine Fusionsprotein, das aus einem Gewebefaktor bindenden Protein besteht, welches an ein EGF456 Fragment des Thrombomodulins gebunden ist, welches alleine oder in Kombination mit mindestens einer aus der folgenden Gruppe der TM-Domänen vorkommt, bestehend aus der N-terminalen hydrophoben Domäne, der EGF123-Domänene, der Interdomänenschleife zwischen EGF3 und EGF4, und der O-glykosylierten Serin/Threonin-reichen Domäne oder Analoga, Fragmente oder Derivate hiervon. Die Patentschrift US 6 632 791 beansprucht Thrombomodulinanaloga, die Thrombin-vermittelt Protein C aktivieren können, allerdings eine signifikant verringerte Aktivierung des Thrombin-aktivierbaren Fibrinolyse Inhibitors (TAFI) aufweisen. Dies wurde durch Austausch einer der drei Aminosäuren Valin340, Aspartat 341 oder Glutamat 343 gegen Alanin in Verbindung mit einem Austausch Histidin381 gegen Glycin erreicht. In einer weiteren Ausführungsform kann ein Kaninchen-Thrombomodulin benutzt werden.

Die Konzentration des löslichen oder zellgebundenen Thrombomodulins kann mit Hilfe von Thrombomodulin-bindenden Substanzen, bevorzugt mit monoklonalen anti-Thrombomodulin Antikörpern, besonders bevorzugt mit den monoklonalen anti-Thrombomodulin Antikörpern ZM43b und TM531 bestimmt werden. Antikörper ZM43B (auch unter den Bezeichnungen TM 43B und 71-43B bekannt) ist erhältlich von American Diagnostica Inc., Stamford, CT, Artikelnr. 2375). Der Antikörper TM531 ist beschrieben in Bajzar et al., "Both Cellular and Soluble Forms of Thrombomodulin Inhibit Fibrinolysis by Potentiating the Activation of Thrombin-activable Fibrinolysis Inhibitor." J. Biol. Chem. 273:2792-2798.

Weitere Beispiele für mögliche TM-bindende Substanzen sind Lektine, Aptamere, Ribozyme, chimäre Antikörper, Einzelketten-Antikörper oder Nanobodies.

Die vorliegende Erfindung kann auch zur Identifizierung weiterer Risikofaktoren bei den zu behandelnden oder beratenden Individuen, seien es Probanden oder Patienten, verwendet werden.

Für die Zwecke der vorliegenden Beschreibung werden die Begriffe wie folgt verwendet:

Gerinnungshemmer im Sinne der vorliegenden Erfindung sind Stoffe, die entweder als Hauptwirkung oder als Nebenwirkung eine Hemmung oder Reduktion der Blutgerinnung bewirken. Zu den Medikamenten mit einer solchen Hauptwirkung zählen die direkten Antikoagulantien, die indirekten Antikoagulantien und die Thrombozytenfunktionshemmer. Zu den Medikamenten, die diese ??? als Nebenwirkung auslösen, zählen beispielsweise nichtsteroidale Entzündungshemmer wie Indomethacin, Phenylbutazon, Naproxen, Ibuprofen oder Fenoprofen, selektive Serotonin-Reuptake Hemmer (SSRI) wie Fluoxetin, Fluvoxamin, Paroxetin, Sertralin, Citalopram oder Escitalopram oder Thrombolytika wie Ancrod, Streptokinase, Urokinase, Alteplase (rt-PA), Reteplase (r-PA), Tenecteplase (TNK-tPA) oder Desmoteplase (DSPA).

Antikoagulantien sind Stoffe, die die Gerinnungsfähigkeit des Blutes verringern oder aufheben. Man unterscheidet direkte Antikoagulantien und indirekte Antikoagulantien (siehe unten). Antikoagulantien werden bevorzugt zur Hemmung venöser Thrombosen eingesetzt. Diese Thrombosen entstehen in Bereichen mit geringer Blutflussgeschwindigkeit bevorzugt in den Venen der unteren Körperhälfte (tiefe Beinvenen, nicht selten auch Beckenvenen).

Direkte Antikoagulantien interagieren direkt mit den Gerinnungsfaktoren. Beispiele hierfür sind Heparin, Heparinoide als halbsynthetische Abkömmlinge, Hirudin, Faktor X Inhibitoren, und direkte Thrombininhibitoren.

Indirekte Antikoagulantien hemmen oder reduzieren die Biosynthese der Gerinnungsfaktoren. Hierzu zählen 4-Hydroxycumarin und dessen Derivate (Dicumarol, Marcumar (Phenprocoumon), Warfarin). Diese Substanzen wirken als Vitamin-K-Antagonisten und werden wegen ihrer geringen therapeutischen Breite nur unter strenger Überwachung eingesetzt.

Vitamin K-Antagonisten hemmen oder reduzieren die Vitamin K Epoxid-Reduktase, die bei der Vitamin K-abhängigen Carboxylierungsreaktion notwendig ist. Eine Reihe von Gerinnungsfaktoren wie Faktor II, VII, IX, X, Protein C und Protein S werden über die Carboxylierung von Glutamylresten aktiviert, da diese dann in Gegenwart von Calciumionen an Phospholipidmembranen gebunden werden. Damit hat Vitamin K eine wesentliche Funktion in der Blutgerinnung. Gerinnungshemmende Medikamente der Cumarin-Gruppe wie Phenprocoumon oder Warfarin können durch vergleichsweise kleine Mengen Vitamin K (1 mg) in ihrer Wirkung aufgehoben werden; sind sie im Einsatz, darf kein Vitamin K zusätzlich zur normalen Nahrung aufgenommen werden.

Die Bezeichnung "Warfarin" umfasst Warfarinderivate mit antikoagulativer Aktivität. Eine bevorzugte Ausführung ist Warfarin als 4-Hydroxy-3-(3-oxo-1-phenylbutyl)-2H-1-Benzopyran-2-on (entspricht dem 3-α-Pheneyl-β-Acetylethyl-4-Hydroxycumarin). Das auf dem Markt befindliche Produkt ist ein racemisches Gemisch aus dem S-Isomer und dem R-Isomer. Die Bezeichnung "Warfarin" umfasst das R-Isomer, das S-Isomer, jegliches razemisches Gemisch und die Salze hiervon. Spezifisch eingeschlossen als Warfarin sind Coumadin, Marevan, Panwarfin, Prothramidon, Tintorane, Warfilone, Waran, Athrombin-K, warfarin-deanol, Adoisine, Warfarinsäure, Coumafene, Zoocoumarin, Phenprocoumon, dicumarol, Brodifacoum, diphenadione, chlorophacinone, bromadiolone und aceno-coumarol.

Thrombozytenfunktionshemmer hemmen oder reduzieren die Aktivierung oder Aggregation der Thrombozyten, die als Vorgang der zellulären Hämostase dem Verschluß von Blutgefäßen dient. Acetylsalicylsäure (Aspirin) kann in die Thrombozytenaggregation, also in die zelluläre Hämostase, eingreifen. Eine Cyclooxygenase (COX), die für die Synthese des Plättchenfaktors Thromboxan A₂ benötigt wird, wird irreversibel durch Anheftung eines Essigsäure-Restes gehemmt. Ebenfalls auf die Aggregation der Thrombozyten wirkt Clopidogrel, das eine Hemmung der ADP-abhängigen Thrombozytenaktivierung durch eine irreversible Rezeptor-Blockierung bewirkt. Abciximab ist ein rekombianter monoklonaler Antikörper, der das Glykoprotein IIb/IIIa der Thrombozyten blockiert und dadurch gleichfalls die Thrombozytenaggregation unterbindet. Denselben Angriffsort besitzt auch Tirofiban.

### Ausführungsbeispiel

Untersuchung der Konzentration an löslichem Thrombomodulin in Patienten, die mit Warfarin behandelt werden.

### 1. Testsystem

In einer prospektiven Kohortenstudie wurden 719 Patienten unter Warfarinbehandlung über eine mittlere Zeit von 4.2 Jahren verfolgt. Sämtliche Blutungskomplikationen, die zu einer Krankenhauseinweisung führten, wurden erfasst und in klinisch relevante Blutungen und starke Blutungen klassifiziert. Ischämische Schlaganfälle, periphere arterielle Thrombi, Myokardinfarkte und Todesfälle wurden ebenfalls erfasst und klassifiziert. Das lösliche Thrombomodulin Antigen wurde mit einer ELISA Methode bestimmt.

### 2. Experimentelle Methoden

### Patienten

In einer prospektiven Kohortenstudie wurde 719 Patienten unter Behandlung mit Vitamin K Antagonisten (hiervon über 96% mit Warfarin behandelt) eingeschlossen. Die Patienten wurden im Juni 1996 rekrutiert. Eine Vorbehandlung mit Warfarin über mehr als 3 Monate wurde als Langzeitbehandlung definiert und diente als Einschlusskriterium.

### Blutentnahme und Laborarbeiten

Venöses Blut wurde bei minimierter Blutstauung entnommen und in silikonisierten Routine-Citratplasmaröhrchen abgefüllt, die 0.13mol/L Natriumcitrat enthalten. Nach Zentrifugation wurden die Plasmaproben eingefroren und bis zur Analyse bei -70°C gelagert.

Die Messung des löslichen Thrombomodulins im Plasma wurde über eine ELISA-Methode bestimmt.

Die Intraassay- und Interassay-Variabilität lag unter 5% beziehungsweise 6%. Das ultrasensitive C-reaktive Protein (high sensitivity C-reactive protein; hsCRP) wurde mit einer automatisierten hsCRP Methode bestimmt (Immulite Diagnostics Products Corporation, USA). Die Interassay-Variabilität lag unter 6%. Die Prothrombinzeit wurde als INR (international normalized ratio) in den jeweiligen Zentrallaboratorien der Kliniken bestimmt.

### Protokoll der Anschlußstudie

Alle Patienten wurden weiterverfolgt bis zu einem Blutungsereignis, dem Absetzen der VKA-Behandlung, dem Tod oder dem 1. Januar 2002 als Endzeitpunkt der Studie. Die maximale Verweildauer in der Studie betrug 5.6 Jahre, die durchschnittliche Verweildauer 4.2 Jahre.

Um alle Blutungsereignisse zu identifizieren, die zur Aufnahme in das Krankenhaus oder zum Tode führten, wurden alle Krankenprotokolle von den Abteilungen für Innere Medizin, Chirurgie, Hals-, Nasen- und Ohrenheilkunde, Augenheilkunde, Urologie, Neurologie, Neurochirurgie und orthopädischer Chirurgie für den Zeitraum vom 1. Juni 1996 bis zum 1. Januar 2002 gesichtet. Ein Patient verließ während der Studienzeitraums die Region und wurde bis zum Zeitpunkt des Umzugs nachverfolgt. Bei 136 Patienten wurde während der Studie die Warfarin-Behandlung abgesetzt. Alle Blutungsereignisse, die zur Aufnahme in das Krankenhaus oder zum Tode führten, wurden erfasst und durch eine Gruppe von 3 Forschern (Marcus Lind, Jan-Hakan Jansson und Lars Johansson) unabhängig voneinander klassifiziert. Schwere Blutungen wurden nach Schulman und Kearon definiert als tödliche Blutungen und/oder symptomatische Blutungen in einem kritischen Bereich oder Organ und/oder Blutungen, die mit einer Abnahme des Hämoglobinspiegels um 20g/L oder mehr einhergingen oder die zu Bluttransfusionen von zwei oder mehr Einheiten Vollblut führten (Schulman S & Kearon C: "Definition of major bleeding in clinical investigations of antihemostatic medicinal products in non-surgical patients.", J Thromb Haemost 2005: 3(4): 692-694). Klinisch relevante Blutungen wurden definiert als schwere Blutungen oder offenkundige Blutungen, die basierend auf objektiven Untersuchungen zur Aufnahme in ein Krankenhaus oder einem verlängertem Krankenhausaufenthalt führten, aber nicht den Kriterien für schwere Blutungen aufwiesen. Alle übrigen Blutungsereignisse wurden als geringfügige Blutungen klassifiziert und von der Analyse ausgeschlossen. Myokardinfarkte, ischämische Schlaganfälle und periphere arterielle Emboli wurden erfasst. Die jeweiligen Todesursachen wurden verzeichnet und anhand der Sterbeurkunde klassifiziert. Mit Ausnahme eines Patienten konnten alle Todesfälle auf diese Weise klassifiziert werden. Die durch die Prüfer klassifizierten Ereignisse wurden für die biochemische Analyse verblindet.

### Statistische Analyse

Um die Signifikanz der Unterschiede im arithmetischen Mittel und den Größenverhältnissen zu ermitteln, wurde der Mann-Whitney Test und der Chi-Quadrat Test angewendet. Der Spearman-Korrelationskoeffizient wurde verwendet, um mögliche Abhängigkeiten zwischen den Variabeln zu ermitteln. Die Analyse des löslichen Thrombomodulins wurde als kontinuierliche Variable durchgeführt und die Daten in Tertilen kategorisiert, wobei die niedrigste Tertile als Referenzgruppe fungierte. Die Verteilung des sTM und der hsCRP wurde aufgrund der ungleichmäßigen Verteilung logarithmisch transformiert (natürlicher Logarithmus In). Für jede Variable wurde eine univariate Cox-Regressionsanalyse durchgeführt, um die Hazardwerte (hazard ratio, HR) und das 95%-Konfidenzintervall (CI) abzuschätzen. Da die durch diese univariaten Analysen gefundenen Faktoren aber potentiell miteinander zusammenhängen können, wurden multivariate Cox-Regressionen durchgeführt, um voneinander unabhängige Prognosefaktoren identifizieren zu können. Faktoren wurden von der multivariaten Analyse ausgeschlossen, wenn die univariate Cox-Regression einen klar nicht-signifikanten Hazardwert ergab (p> 0.2). Überlebenszeitfunktionen mit dem Anteil an Patienten ohne schwere Blutungen wurden berechnet. Die aus dem Skellefteå Bezirkskrankenhaus vorliegenden Daten über Diabetes, frühere Magengeschwüre, Bluthochdruck und den Bodymass Index (BMI) wurden mit Hilfe der univariaten Cox-Analyse auf eine mögliche Korrelation mit Blutungsereignissen getestet.

Ein P-Wert von < 0.05 wurde als statistisch signifikant erachtet. Sämtliche statistischen Analysen wurden mit dem Programm SPSS (SPSS Inc., Version 15.0) erstellt. Mit 73 schweren Blutungen und 643 ereignisfreien Kontrollen und der statistischen Power von 80%, wurden ORs mit < 0.44 oder >2.0 bei einem 5% Signifikanzniveau von 5% und einer Prävalenz von 33% als signifikant berechnet. Individuen mit fehlenden Werten (30 für sTM und 2 für hsCRP) wurden von der statistischen Analyse ausgeschlossen

### 3. Ergebnisse

Die klinische Ausgangsituation und die Indikationen, die zur Warfarin-Behandlung führten, sind in Tabelle 1 dargestellt. Das durchschnittliche Alter der Patienten betrug 70 Jahre, der Anteil der weiblichen Patienten betrug 37%. Die häufigste Indikation für eine Warfarin-Behandlung war Vorhofflimmern, gefolgt von prothetischen Herzklappen. Die Nachverfolgungszeit betrug 3001 Behandlungsjahre in 719 Patienten. Insgesamt wurden 113 klinisch relevante Blutungen und 73 schwere Blutungen verzeichnet. Die klinisch relevanten Blutungen waren hauptsächlich mit 37% gastrointestinal bedingt, gefolgt von intrakraniellen Blutungen mit 16% und bindegeweblichen Blutungen mit 13%. Tödliche Blutungen traten in 11 Patienten auf (0.4% pro Behandlungsjahr). Das gemessene lösliche Thrombomodulin wurde in Tertilen eingeteilt; unter 5.0 ng/ml, 5.1-6.1 ng/ml und mehr als 6.1 ng/ml.

**Tabelle 1. Grundeigenschaften der Studienkohorte**

| | **Studienkohorte (n=719)** |
|---|---|
| Alter bei Einschluss in die Studie | 70 ± 11 |
| Frauen (%) | 268 (37) |
| Durchschnittliche Verfolgungszeit, Jahre | 4.2 ± 1.8 |
| sTM-Spiegel, ng/mL | 6.1 ± 2.8 |
| HsCRP-Spiegel, mg/mL | 7.0 ± 14.9 |
| Indikationen für Warfarin-Behandlung: | |
| - Prosthetische Herzklappe (%) | 248 (35) |
| - Herzflimmern (%) | 228 (32) |
| - Periphere arterielle | 40 (6) |
| Thromboembolien (%) | |
| - Ischämischer Schlaganfall (%) | 73 (10) |
| - Venöse Thrombose (%) | 83 (11) |
| - Sonstige Indikationen (%) | 40 (6) |
| - nicht definiert (%) | 7 (1) |

### Blutungsereignisse

Das jährliche Risiko für klinisch relevante Blutungen betrug 3.9% und für schwere Blutungen 2.4%. sTM korrelierte signifikant mit dem hsCRP-Spiegel (r=0.15) und dem Alter (r=0.23). 24% der klinischen relevanten Blutungen und 27% der schweren Blutungen traten bei INR-Werten >3.5 auf. Die univariate Analyse zeigte, dass das Alter, sTM als kontinuierliche Variable und die zweite und dritte Tertile des sTM-Spiegels signifikant mit klinischen Blutungen und schweren Blutungen assoziiert waren. Sowohl das Geschlecht, als auch der hsCRP-Spiegel waren nicht mit dem Risiko für klinische Blutungen und schwere Blutungen verknüpft (siehe Tabelle 2). Ebenfalls nicht korreliert mit den Blutungen waren die klinischen Parameter Bluthochdruck, Diabetes, BMI, vorherige Magen- oder Zwölffingerdarmgeschwüre mit und ohne Blutungsgeschehen.

**Tabelle 2. Univariate und multivariate Cox-Regressionsanalyse zur Ermittlung des Hazardwertes (95% Konfidenzinterval) für die klinisch relevanten Blutungen und schweren Blutungen.**

| | Klinisch relevante Blutungen | | Schwere Blutungen | |
|---|---|---|---|---|
| | Univariat | Multivariat | Univariat | Multivariat |
| Alter (pro Jahr) | 1.04 (1.02-1.07) | 1.04 (1.01-1.06)¹ | 1.04 (1.01-1.06) | 1.03 (1.00-1.05)¹ |
| Frauen | 1.09 (0.75-1.59) | | 1.31 (0.82-2.08) | |
| sTM kontinuierlic h sTM | 2.81 (1.77-4.46) | 2.52 (1.54-4.11)² | 3.20 (1.83-5.58) | 2.97(1.66-5.31)² |
| 1. Tertile (n=221) | 1.0 | 1.0 | 1.0 | 1.0 |
| 2. Tertile (n=241) | 2.11 (1.24-3.59) | 1.86 (1.09-3.19)¹ | 1.99 (1:02-3.88) | 1.83 (0.94-3.57)¹ |
| 3. Tertile3(n=2 27) | 2.74 (1.63-4.61) | 2.32 (1.36-3.93)¹ | 2.69 (1.41-5.14) | 2.37 (1.23-4.56)¹ |
| HsCRP kontinuierlich | 1.01 (0.85-1.20) | | 1.00 (0.81-1.24) | |

| | | | | |
|---|---|---|---|---|
| Abkürzungen: sTM - Lösliches Thrombomodulin Antigen, hsCRP - ultrasensitives C-reaktives Protein. | | | | |

Das multivariate Modell beinhaltete Alter und die sTM-Tertilen oder Alter und den sTM-Spiegel als kontinuierliche Variable. STM und hsCRP wurden log-transformiert.

Das multivariate Modell zeigte, dass Alter, sTM als kontinuierliche Variable und die dritte Tertile des sTM-Spiegels signifikant sowohl mit den klinisch relevanten Blutungen, als auch mit den schweren Blutungen korrelierten. Die altersbereinigte Inzidenz klinisch relevanter Blutungen pro 100 Behandlungsjahre betrug 2.5 in der niedrigsten Tertile, 4.1 in der mittleren Tertile und 4.9 in der höchsten Tertile. Die korrespondierende Inzidenz schwerer Blutungen pro 100 Behandlungsjahre betrug 1.5 in der niedrigsten Tertile, 2.4 in der mittleren Tertile und 3.7 in der höchsten Tertile.

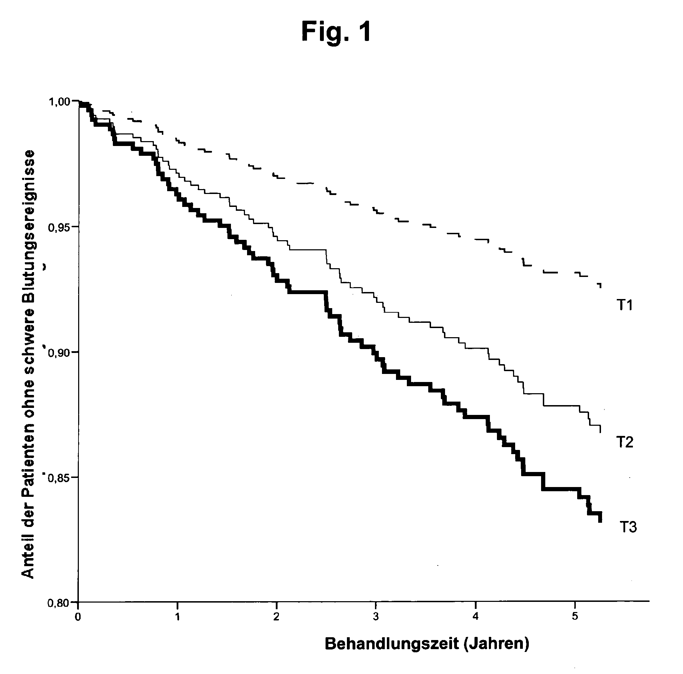

Altersbereinigte Überlebenskurven verdeutlichen den unterschiedlichen Anteil an Patienten ohne schwere Blutungen in den verschieden Tertilen des sTM Spiegels (siehe Abbildung 1). Eine mögliche Wechselbeziehung zwischen Alter und sTM-Spiegel wurde anhand der klinisch relevanten Blutungen als Ergebnisparameter untersucht. Ältere Patienten (70 Jahre und älter) mit hohem sTM-Spiegel (über dem arithmetischem Mittel von 5.61ng/mL) besaßen im Vergleich zu den als Kontrollen verwendeten jüngeren Patienten (Alter <70 Jahre) mit niedrigem sTM-Spiegel (unterhalb des arithmetischen Mittels) einen Hazardwert von 3.7. Der Hazardwert für das kombinierte Auftreten von höherem Alter und niedrigem sTM-Spiegel lag bei 2.4 im Vergleich zu den Kontrollen. Der Hazardwert für das kombinierte Auftreten von niedrigem Alter und höherem sTM-Spiegel lag bei 1.6 im Vergleich zu den Kontrollen. Alter und sTM-Spiegel zeigten keinen synergistischen Effekt.

Die Stratifizierung nach Geschlecht zeigte, dass Frauen mit sTM-Spiegeln in der dritten Tertile im Vergleich zur ersten Tertile (p=0.01) einen Hazardwert von 3.27 (95% Cl, 1.37 bis 7.79) im Hinblick auf klinisch relevante Blutungen besaßen. Die Altersbereinigte Inzidenz klinisch relevanter Blutungen pro 100 Behandlungsjahren betrug 2.1% in der niedrigsten Tertile und 5.6% in der höchsten Tertile. In Männern betrug der Hazardwert für klinisch relevante Blutungen 1.85 (95% Cl, 0.95 bis 3.63). Die Stratifizierung bezüglich der beiden beteiligten Warfarin-Kliniken ergab altersbereinigt eine signifikante Beziehung zwischen dem sTM-Spiegel und klinisch relevanten Blutungen in beiden Populationen; der Hazardwert der dritten Tertile betrug im Vergleich zu ersten Tertile 2.34 in Skellefteå und 2.52 in Umeä.

### Nichttödliche kardiovaskuläre Ereignisse und Gesamttodesfälle

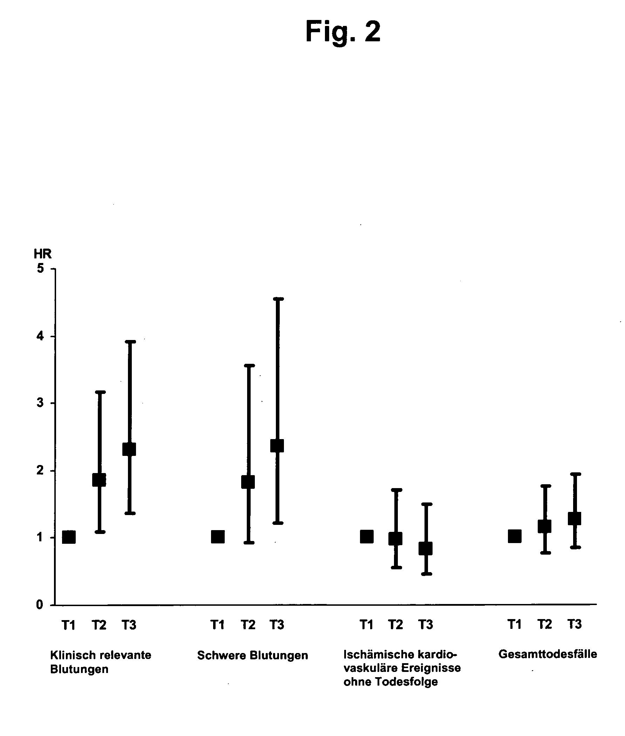

Im Verlauf der Warfarin-Behandlung starben insgesamt 161 Patienten. Die häufigste Todesursache war der Herzinfarkt gefolgt von Schlaganfall. Im Ganzen traten 96 kardiovaskulär bedingte Todsfälle und 75 kardiovaskuläre Ereignisse ohne Todesfolge auf. Die univariate Analyse zeigte eine signifikante Korrelation des Alters mit den Gesamttodesfällen (HR von 1.06; 95% Cl, 1.04 bis 1.08) und mit den ischämischen kardiovaskulären Ereignissen ohne Todesfolge (HR von 1.05; 95% Cl, 1.02 bis 1.07). Als wichtiger Befund gilt hervorzuheben, dass keine signifikante Korrelation zwischen dem sTM-Spiegeln und Gesamttodesfällen oder den ischämischen kardiovaskulären Ereignissen ohne Todesfolge gefunden werden konnte. Die Abbildung 2 zeigt die altersbereinigten Regressionsanalysen für die sTM-Tertilen in Bezug auf die klinisch relevanten Blutungen, die schweren Blutungen, die Gesamttodesfälle und die kardiovaskulären Ereignisse ohne Todesfolge.

### 4. Legenden

**Fig. 1****.** Altersbereinigte Überlebenskurven, die für die drei Tertilen T1-T3 des löslichen Thrombomodulins den Anteil der Patienten ohne schwere Blutungsereignisse angeben.
**Fig. 2****.** Altersbereinigte Hazardwerte (HR) für klinisch relevante Blutungen, schwere Blutungen, kardiovaskuläre Ereignisse ohne Todesfolge und Gesamttodesfälle für die jeweiligen Tertilen (T1-T3) des löslichen Thrombomodulins.
**Fig. 3****.** Schematische Übersicht zur Entscheidung über eine Antikoagulanztherapie anhand des Spiegels an löslichem Thrombomodulin
   Sequenz ID No. 1. Aminosäuresequenz des humanen Thrombomodulins
   Sequenz ID No. 2. Nukleotidsequenz der kodierenden Region des Solulins

## Patentansprüche

1. In vitro-Verfahren zur Auswahl eines für die Therapie mit Gerinnungshemmern geeigneten Patienten, umfassend folgende Schritte:
a. Entnahme einer Probe aus einer Körperflüssigkeit, -zellen oder -gewebe aus dem Patienten,
b. Ermittlung des löslichen Thrombomodulins in der entnommenen Probe,
c. Vergleich der ermittelten Konzentration oder Aktivität des löslichen Thrombomodulins in der Probe mit einem Standardwert,
d. Auswahl desjenigen Patienten, dessen Probe im Vergleich zu dem Standardwert keine wesentlich erhöhte Konzentration oder Aktivität an löslichem Thrombomodulin aufweist für eine Behandlung mit Gerinnungshemmern.

2. Verfahren nach Anspruch 1, **dadurch gekennzeichnet, dass** der Standartwert
a) dem arithmetischen Mittel der unteren Tertile einer Kontrollgruppe oder
b) etwa 80 % des aritmetischen Mittels aller Werte einer Konrollgruppe entspricht.

3. Verfahren nach Anspruch 1, **dadurch gekennzeichnet, dass** der ausgewählte Patient eine Konzentration von löslichem Thrombomodulin im Blutplasma unter etwa 6.0 ng/ml, bevorzugt unter etwa 5,5 ng/ml und besonders bevorzugt unter etwa 5,0 ng/ml besitzt.

4. Verfahren nach einem der vorherigen Ansprüche, **dadurch gekennzeichnet, dass** der Patient für eine Therapie mit Antikoagulantien, Thrombolytika oder Blutplättchenaggregationshemmer ausgewählt wird.

5. Verfahren nach Anspruch 4, **dadurch gekennzeichnet, dass** der Patient für eine Therapie mit indirekten Antikoagulantien, bevorzugt mit Vitamin K-Antagonisten, besonders bevorzugt mit Warfarin ausgewählt wird.

6. Verfahren nach einem der vorherigen Ansprüche, **dadurch gekennzeichnet, dass** das die Therapie der vorbeugenden Behandlung einer thromboembolischen Erkrankung dient.

7. Verfahren nach einem der vorherigen Ansprüche, **dadurch gekennzeichnet, dass** die Konzentration oder Aktivität des löslichen Thrombomodulins mit Hilfe von Thrombomodulin-bindenden Substanzen, bevorzugt mit monoklonalen anti-Thrombomodulin Antikörpern, besonders bevorzugt mit den monoklonalen anti-Thrombomodulin Antikörpern ZM43b und TM531 bestimmt wird.

8. Kit umfassend mindestens eine Thrombomodulin-bindende Substanz sowie lösliches Thrombomodulin, bevorzugt humanes lösliches Thrombomodulin, besonders bevorzugt Solulin.

9. Verfahren zur Bestimmung des löslichen Thrombomodulins in einer Probe, **dadurch gekennzeichnet, dass** das Thrombomodulin in einem ersten Schritt durch einen monoklonalen Anti-Thrombomodulin-Antikörper als Nachweis-Antikörper nachgewiesen wird, wobei der Nachweis-Antikörper nicht mit dem Capture-Antikörper interferriert.

10. Verwendung des Werts für die Menge oder Aktivität des löslichen Thrombomodulins in einer Probe aus einem Menschen oder Tier als Biomarker für die Eignung dieses Menschen oder Tieres, mit gerinnungshemmenden Mitteln behandelt zu werden, ohne eine klinisch inakzeptable Disposition für ein Blutungsrisiko zu zeigen.


SEQUENCE LISTING

<110> PAION Deutschland GmbH
Oehlin, Ann-Kristin
Gillen, Clemens

<120> Lösliches Thrombomodulin als Biomarker bei Therapien mit
Gerinnungshemmmern

<130> 48709 K

<160> 3

<170> PatentIn version 3.5

<210> 1
<211> 575
<212> PRT
<213> Homo sapiens

<400> 1

Met Leu Gly Val Leu Val Leu Gly Ala Leu Ala Leu Ala Gly Leu Gly
1               5                   10                  15

Phe Pro Ala Pro Ala Glu Pro Gln Pro Gly Gly Ser Gln Cys Val Glu
            20                  25                  30

His Asp Cys Phe Ala Leu Tyr Pro Gly Pro Ala Thr Phe Leu Asn Ala
        35                  40                  45

Ser Gln Ile Cys Asp Gly Leu Arg Gly His Leu Met Thr Val Arg Ser
    50                  55                  60

Ser Val Ala Ala Asp Val Ile Ser Leu Leu Leu Asn Gly Asp Gly Gly
65                  70                  75                  80

Val Gly Arg Arg Arg Leu Trp Ile Gly Leu Gln Leu Pro Pro Gly Cys
                85                  90                  95

Gly Asp Pro Lys Arg Leu Gly Pro Leu Arg Gly Phe Gln Trp Val Thr
            100                 105                 110

Gly Asp Asn Asn Thr Ser Tyr Ser Arg Trp Ala Arg Leu Asp Leu Asn
        115                 120                 125

Gly Ala Pro Leu Cys Gly Pro Leu Cys Val Ala Val Ser Ala Ala Glu
    130                 135                 140

Ala Thr Val Pro Ser Glu Pro Ile Trp Glu Glu Gln Gln Cys Glu Val
145                 150                 155                 160

Lys Ala Asp Gly Phe Leu Cys Glu Phe His Phe Pro Ala Thr Cys Arg
                165                 170                 175

Pro Leu Ala Val Glu Pro Gly Ala Ala Ala Ala Ala Val Ser Ile Thr
            180                 185                 190

Tyr Gly Thr Pro Phe Ala Ala Arg Gly Ala Asp Phe Gln Ala Leu Pro
        195                 200                 205

Val Gly Ser Ser Ala Ala Val Ala Pro Leu Gly Leu Gln Leu Met Cys
    210                 215                 220

Thr Ala Pro Pro Gly Ala Val Gln Gly His Trp Ala Arg Glu Ala Pro
225                 230                 235                 240

Gly Ala Trp Asp Cys Ser Val Glu Asn Gly Gly Cys Glu His Ala Cys
                245                 250                 255

Asn Ala Ile Pro Gly Ala Pro Arg Cys Gln Cys Pro Ala Gly Ala Ala
            260                 265                 270

Leu Gln Ala Asp Gly Arg Ser Cys Thr Ala Ser Ala Thr Gln Ser Cys
        275                 280                 285

Asn Asp Leu Cys Glu His Phe Cys Val Pro Asn Pro Asp Gln Pro Gly
    290                 295                 300

Ser Tyr Ser Cys Met Cys Glu Thr Gly Tyr Arg Leu Ala Ala Asp Gln
305                 310                 315                 320

His Arg Cys Glu Asp Val Asp Asp Cys Ile Leu Glu Pro Ser Pro Cys
                325                 330                 335

Pro Gln Arg Cys Val Asn Thr Gln Gly Gly Phe Glu Cys His Cys Tyr
            340                 345                 350

Pro Asn Tyr Asp Leu Val Asp Gly Glu Cys Val Glu Pro Val Asp Pro
        355                 360                 365

Cys Phe Arg Ala Asn Cys Glu Tyr Gln Cys Gln Pro Leu Asn Gln Thr
    370                 375                 380

Ser Tyr Leu Cys Val Cys Ala Glu Gly Phe Ala Pro Ile Pro His Glu
385                 390                 395                 400

Pro His Arg Cys Gln Met Phe Cys Asn Gln Thr Ala Cys Pro Ala Asp
                405                 410                 415

Cys Asp Pro Asn Thr Gln Ala Ser Cys Glu Cys Pro Glu Gly Tyr Ile
            420                 425                 430

Leu Asp Asp Gly Phe Ile Cys Thr Asp Ile Asp Glu Cys Glu Asn Gly
        435                 440                 445

Gly Phe Cys Ser Gly Val Cys His Asn Leu Pro Gly Thr Phe Glu Cys
    450                 455                 460

Ile Cys Gly Pro Asp Ser Ala Leu Val Arg His Ile Gly Thr Asp Cys
465                 470                 475                 480

Asp Ser Gly Lys Val Asp Gly Gly Asp Ser Gly Ser Gly Glu Pro Pro
                485                 490                 495

Pro Ser Pro Thr Pro Gly Ser Thr Leu Thr Pro Pro Ala Val Gly Leu
            500                 505                 510

Val His Ser Gly Leu Leu Ile Gly Ile Ser Ile Ala Ser Leu Cys Leu
        515                 520                 525

Val Val Ala Leu Leu Ala Leu Leu Cys His Leu Arg Lys Lys Gln Gly
    530                 535                 540

Ala Ala Arg Ala Lys Met Glu Tyr Lys Cys Ala Ala Pro Ser Lys Glu
545                 550                 555                 560

Val Val Leu Gln His Val Arg Thr Glu Arg Thr Pro Gln Arg Leu
                565                 570                 575

<210>  2
<211>  1518
<212>  DNA
<213>  Homo sapiens

<220>
<221>  CDS
<222>  (1)..(1515)
<223>  Solulin protein including signal peptide

<220>
<221>  mat_peptide
<222>  (55)..()

<400>  2
atg ctt ggg gtc ctg gtc ctt ggc gcg ctg gcc ctg gca ggc ctg gtc      48
Met Leu Gly Val Leu Val Leu Gly Ala Leu Ala Leu Ala Gly Leu Val
            -15                 -10                 -5

ttc ccc gag ccg cag ccg ggt ggc agc cag tgc gtc gag cac gac tgc      96
Phe Pro Glu Pro Gln Pro Gly Gly Ser Gln Cys Val Glu His Asp Cys
    -1  1               5                   10

ttc gcg ctc tac ccg ggc ccc gcg acc ttc ctc aat gcc agt cag atc      144
Phe Ala Leu Tyr Pro Gly Pro Ala Thr Phe Leu Asn Ala Ser Gln Ile
15                  20                  25                  30

tgc gac gga ctg cgg ggc cac cta atg aca gtg cgc tcc tcg gtg gct      192
Cys Asp Gly Leu Arg Gly His Leu Met Thr Val Arg Ser Ser Val Ala
                35                  40                  45

gcc gat gtc att tcc ttg cta ctg aac ggc gac ggc ggc gtt ggc cgc      240
Ala Asp Val Ile Ser Leu Leu Leu Asn Gly Asp Gly Gly Val Gly Arg
            50                  55                  60

cgg cgc ctc tgg atc ggc ctg cag ctg cca ccc ggc tgc ggc gac ccc      288
Arg Arg Leu Trp Ile Gly Leu Gln Leu Pro Pro Gly Cys Gly Asp Pro

65                  70                  75

aag cgc ctc ggg ccc ctg cgc ggc ttc cag tgg gtt acg gga gac aac      336
Lys Arg Leu Gly Pro Leu Arg Gly Phe Gln Trp Val Thr Gly Asp Asn
    80                  85                  90

aac acc agc tat agc agg tgg gca cgg ctc gac ctc aat ggg gct ccc      384
Asn Thr Ser Tyr Ser Arg Trp Ala Arg Leu Asp Leu Asn Gly Ala Pro
95                  100                 105                 110

ctc tgc ggc ccg ttg tgc gtc gct gtc tcc gct gct gag gcc act gtg      432
Leu Cys Gly Pro Leu Cys Val Ala Val Ser Ala Ala Glu Ala Thr Val
                115                 120                 125

ccc agc gag ccg atc tgg gag gag cag cag tgc gaa gtg aag gcc gat      480
Pro Ser Glu Pro Ile Trp Glu Glu Gln Gln Cys Glu Val Lys Ala Asp
            130                 135                 140

ggc ttc ctc tgc gag ttc cac ttc cca gcc acc tgc agg cca ctg gct      528
Gly Phe Leu Cys Glu Phe His Phe Pro Ala Thr Cys Arg Pro Leu Ala
        145                 150                 155

gtg gag ccc ggc gcc gcg gct gcc gcc gtc tcg atc acc tac ggc acc      576
Val Glu Pro Gly Ala Ala Ala Ala Ala Val Ser Ile Thr Tyr Gly Thr
    160                 165                 170

ccg ttc gcg gcc cgc gga gcg gac ttc cag gcg ctg ccg gtg ggc agc      624
Pro Phe Ala Ala Arg Gly Ala Asp Phe Gln Ala Leu Pro Val Gly Ser
175                 180                 185                 190

tcc gcc gcg gtg gct ccc ctc ggc tta cag cta atg tgc acc gcg ccg      672
Ser Ala Ala Val Ala Pro Leu Gly Leu Gln Leu Met Cys Thr Ala Pro
                195                 200                 205

ccc gga gcg gtc cag ggg cac tgg gcc agg gag gcg ccg ggc gct tgg      720
Pro Gly Ala Val Gln Gly His Trp Ala Arg Glu Ala Pro Gly Ala Trp
            210                 215                 220

gac tgc agc gtg gag aac ggc ggc tgc gag cac gcg tgc aat gcg atc      768
Asp Cys Ser Val Glu Asn Gly Gly Cys Glu His Ala Cys Asn Ala Ile
        225                 230                 235

cct ggg gct ccc cgc tgc cag tgc cca gcc ggc gcc gcc ctg cag gca      816
Pro Gly Ala Pro Arg Cys Gln Cys Pro Ala Gly Ala Ala Leu Gln Ala
    240                 245                 250

gac ggg cgc tcc tgc acc gca tcc gcg acg cag tcc tgc aac gac ctc      864
Asp Gly Arg Ser Cys Thr Ala Ser Ala Thr Gln Ser Cys Asn Asp Leu
255                 260                 265                 270

tgc gag cac ttc tgc gtt ccc aac ccc gac cag ccg ggc tcc tac tcg      912
Cys Glu His Phe Cys Val Pro Asn Pro Asp Gln Pro Gly Ser Tyr Ser
                275                 280                 285

tgc atg tgc gag acc ggc tac cgg ctg gcg gcc gac caa cac cgg tgc      960
Cys Met Cys Glu Thr Gly Tyr Arg Leu Ala Ala Asp Gln His Arg Cys
            290                 295                 300

gag gac gtg gat gac tgc ata ctg gag ccc agt ccg tgt ccg cag cgc      1008
Glu Asp Val Asp Asp Cys Ile Leu Glu Pro Ser Pro Cys Pro Gln Arg
        305                 310                 315

tgt gtc aac aca cag ggt ggc ttc gag tgc cac tgc tac cct aac tac      1056
Cys Val Asn Thr Gln Gly Gly Phe Glu Cys His Cys Tyr Pro Asn Tyr
    320                 325                 330

gac ctg gtg gac ggc gag tgt gtg gag ccc gtg gac ccg tgc ttc aga      1104
Asp Leu Val Asp Gly Glu Cys Val Glu Pro Val Asp Pro Cys Phe Arg

335                 340                 345                 350

gcc aac tgc gag tac cag tgc cag ccc ctg aac caa act agc tac ctc      1152
Ala Asn Cys Glu Tyr Gln Cys Gln Pro Leu Asn Gln Thr Ser Tyr Leu
                355                 360                 365

tgc gtc tgc gcc gag ggc ttc gcg ccc att ccc cac gag ccg cac agg      1200
Cys Val Cys Ala Glu Gly Phe Ala Pro Ile Pro His Glu Pro His Arg
            370                 375                 380

tgc cag ctg ttt tgc aac cag act gcc tgt cca gcc gac tgc gac ccc      1248
Cys Gln Leu Phe Cys Asn Gln Thr Ala Cys Pro Ala Asp Cys Asp Pro
        385                 390                 395

aac acc cag gct agc tgt gag tgc cct gaa ggc tac atc ctg gac gac      1296
Asn Thr Gln Ala Ser Cys Glu Cys Pro Glu Gly Tyr Ile Leu Asp Asp
    400                 405                 410

ggt ttc atc tgc acg gac atc gac gag tgc gaa aac ggc ggc ttc tgc      1344
Gly Phe Ile Cys Thr Asp Ile Asp Glu Cys Glu Asn Gly Gly Phe Cys
415                 420                 425                 430

tcc ggg gtg tgc cac aac ctc ccc ggt acc ttc gag tgc atc tgc ggg      1392
Ser Gly Val Cys His Asn Leu Pro Gly Thr Phe Glu Cys Ile Cys Gly
                435                 440                 445

ccc gac tcg gcc ctt gct ggc cag att ggc acc gac tgt gac tcc ggc      1440
Pro Asp Ser Ala Leu Ala Gly Gln Ile Gly Thr Asp Cys Asp Ser Gly
            450                 455                 460

aag gtg gac ggt ggc gac agc ggc gcc ggc gag ccc ccg ccc agc ccg      1488
Lys Val Asp Gly Gly Asp Ser Gly Ala Gly Glu Pro Pro Pro Ser Pro
        465                 470                 475

acg ccc ggg tct acc ttg act cct ccg tga                              1518
Thr Pro Gly Ser Thr Leu Thr Pro Pro
    480                 485


<210>  3
<211>  505
<212>  PRT
<213>  Homo sapiens

<400>  3

Met Leu Gly Val Leu Val Leu Gly Ala Leu Ala Leu Ala Gly Leu Val
            -15                 -10                 -5


Phe Pro Glu Pro Gln Pro Gly Gly Ser Gln Cys Val Glu His Asp Cys
    -1  1               5                   10


Phe Ala Leu Tyr Pro Gly Pro Ala Thr Phe Leu Asn Ala Ser Gln Ile
15                  20                  25                  30


Cys Asp Gly Leu Arg Gly His Leu Met Thr Val Arg Ser Ser Val Ala
                35                  40                  45


Ala Asp Val Ile Ser Leu Leu Leu Asn Gly Asp Gly Gly Val Gly Arg
            50                  55                  60


Arg Arg Leu Trp Ile Gly Leu Gln Leu Pro Pro Gly Cys Gly Asp Pro
        65                  70                  75

Lys Arg Leu Gly Pro Leu Arg Gly Phe Gln Trp Val Thr Gly Asp Asn
    80                  85                  90

Asn Thr Ser Tyr Ser Arg Trp Ala Arg Leu Asp Leu Asn Gly Ala Pro
95                  100                 105                 110

Leu Cys Gly Pro Leu Cys Val Ala Val Ser Ala Ala Glu Ala Thr Val
                115                 120                 125

Pro Ser Glu Pro Ile Trp Glu Glu Gln Gln Cys Glu Val Lys Ala Asp
            130                 135                 140

Gly Phe Leu Cys Glu Phe His Phe Pro Ala Thr Cys Arg Pro Leu Ala
        145                 150                 155

Val Glu Pro Gly Ala Ala Ala Ala Ala Val Ser Ile Thr Tyr Gly Thr
    160                 165                 170

Pro Phe Ala Ala Arg Gly Ala Asp Phe Gln Ala Leu Pro Val Gly Ser
175                 180                 185                 190

Ser Ala Ala Val Ala Pro Leu Gly Leu Gln Leu Met Cys Thr Ala Pro
                195                 200                 205

Pro Gly Ala Val Gln Gly His Trp Ala Arg Glu Ala Pro Gly Ala Trp
            210                 215                 220

Asp Cys Ser Val Glu Asn Gly Gly Cys Glu His Ala Cys Asn Ala Ile
        225                 230                 235

Pro Gly Ala Pro Arg Cys Gln Cys Pro Ala Gly Ala Ala Leu Gln Ala
    240                 245                 250

Asp Gly Arg Ser Cys Thr Ala Ser Ala Thr Gln Ser Cys Asn Asp Leu
255                 260                 265                 270

Cys Glu His Phe Cys Val Pro Asn Pro Asp Gln Pro Gly Ser Tyr Ser
                275                 280                 285

Cys Met Cys Glu Thr Gly Tyr Arg Leu Ala Ala Asp Gln His Arg Cys
            290                 295                 300

Glu Asp Val Asp Asp Cys Ile Leu Glu Pro Ser Pro Cys Pro Gln Arg
        305                 310                 315

Cys Val Asn Thr Gln Gly Gly Phe Glu Cys His Cys Tyr Pro Asn Tyr
    320                 325                 330

Asp Leu Val Asp Gly Glu Cys Val Glu Pro Val Asp Pro Cys Phe Arg
335                 340                 345                 350

Ala Asn Cys Glu Tyr Gln Cys Gln Pro Leu Asn Gln Thr Ser Tyr Leu
                355                 360                 365

Cys Val Cys Ala Glu Gly Phe Ala Pro Ile Pro His Glu Pro His Arg
            370                 375                 380

Cys Gln Leu Phe Cys Asn Gln Thr Ala Cys Pro Ala Asp Cys Asp Pro
        385                 390                 395

Asn Thr Gln Ala Ser Cys Glu Cys Pro Glu Gly Tyr Ile Leu Asp Asp
    400                 405                 410

Gly Phe Ile Cys Thr Asp Ile Asp Glu Cys Glu Asn Gly Gly Phe Cys
415                 420                 425                 430

Ser Gly Val Cys His Asn Leu Pro Gly Thr Phe Glu Cys Ile Cys Gly
                435                 440                 445

Pro Asp Ser Ala Leu Ala Gly Gln Ile Gly Thr Asp Cys Asp Ser Gly
            450                 455                 460

Lys Val Asp Gly Gly Asp Ser Gly Ala Gly Glu Pro Pro Pro Ser Pro
        465                 470                 475

Thr Pro Gly Ser Thr Leu Thr Pro Pro
    480                 485